**Europäisches Patentamt**

**European Patent Office**

**Office européen des brevets**

(19)

(11) Publication number: **0 046 723**
**A1**

(12) # EUROPEAN PATENT APPLICATION

(21) Application number: **81630049.5**

(22) Date of filing: **11.08.81**

(51) Int. Cl.³: **C 11 C 3/14**
**A 61 K 7/00, A 61 K 9/42**

(30) Priority: **15.08.80 US 177971**
**09.03.81 US 241801**

(43) Date of publication of application:
**03.03.82 Bulletin 82/9**

(84) Designated Contracting States:
**AT BE CH DE FR GB IT LI LU NL SE**

(71) Applicant: **JOJOBA GROWERS & PROCESSORS INC.**
**Madison Avenue 515**
**New York, NY 10022(US)**

(72) Inventor: **Brown, James H.**
**1, Byram Meadow Road**
**Chappaqua, N.Y. 10514(US)**

(72) Inventor: **Olenberg, Harry**
**100-18 Donizetti Place**
**Bronx, N.Y. 10475(US)**

(74) Representative: **Schmitz, Jean-Marie**
**Office Dennemeyer S.à.r.l. 21-25 Allée Scheffer P.O. Box 41**
**Luxembourg(LU)**

(54) **Isomerization of jojoba oil, products thereof and resultant compositions.**

(57) A process for isomerization of natural jojoba oil, a wax-type ester is based on contacting jojoba oil, essentially all cis-configuration with an acidic bentonite-type clay at temperatures in the range 150-350°C. The resulting isomerates, predominately of trans-configuration have melting points in the range 25° to about 44°C.

Also included are useful isomorphous compositions of the trans-isomerates of jojoba oil with hydrogenated jojoba oil. These compositions are useful as candle waxes, non-stick cooking sprays, cosmetic vehicles, carriers for solid "detergent" soaps, lether treatments, hand modifiers for textiles including both woven and non-woven fabrics.

FIG. 1

ISOMERIZATION OF JOJOBA OIL, PRODUCTS THEREOF AND RESULTANT
COMPOSITIONS

This invention relates to jojoba oil modification
and more particularly to the controlled isomerization of
jojoba oil to isomerates having softening points in the
range 25-40°C and suitable for topical application. Also
included are compositions incorporating these isomerates in
combination with hydrogenated jojoba oil.

As set forth in the NRC report of the National
Academy of Science entitled "Products from Jojoba" (1975)
and subsequent publications by Miwa et al, of the Northern
Regional Research Center USDA and Wisniak of Ben Gurion
University as reported in Jojoba Happenings published by
The International Committee of Jojoba Research and Development, jojoba oil is a unique oil and a potential replacement for the sperm whale oil formerly obtained ·from a disappearing and endangered whale species.

Jojoba nut oil differs from most other animal and
vegetable oils in that it is not a fat but a liquid wax.
Fats, including the seed oils of most other plants, are
triglycerides (a mole of glycerol esterified with three
moles of long-chain fatty acids). Jojoba and sperm-oil
are wax esters (one mole of a long-chain alcohol ester-
fied with one mole of a long-chain fatty acid). Jojoba oil
is unique among vegetable oils, as sperm oil is unique among
animal oils. Such a vegetable oil has never before been
available to industry in commercially usable quantities.

The following characteristics make jojoba oil
valuable : its natural purity and molecular simplicity and
its stability. it is a non-drying oil, having high resistance to oxidation, it can be stored for years without becoming
rancid, its lubricity, its unsaturation (double bonds).

The clear, unsaturated oil can be obtained by
pressing or solvent-extraction methods used commercially
to isolate vegetable. oils from cotton seeds, soybeans,
copra and corn.

Such jojoba oil extracts usually have the same
wax-ester composition. The acids and alcohols that make
up these esters do not vary appreciably with location, soil
type, rainfall, or altitude. Moreover, the oil does not change in composition during storage.

Jojoba oil is composed almost entirely of esters of high molecular weight $C_{18-24}$, straight-chain monoethylenic acids and monoethylenic alcohols, for example, erucyl 11-cis-eicosenoate of the formula:

$$CH_3-(CH_2)_7-CH=CH-(CH_2)_{12}-0-CO(CH_2)_9-CH=CH-(CH_2)_7-CH_3$$

These unsaturated acids are a mixture of cis-11-eicosenoic ($C_{20}$) and cis-13-docosenoic ($C_{22}$) erucic, with small quantities of oleic ($C_{18}$) and nervonic ($C_{24}$) acids. The unsaturated alcohols are a mixture of cis-11-eicosenol, cis-13-docosenol and cis-15 tetracosenol, with small quantities of alcohols of lower molecular weight. Note that all the alcohols and acids are primarily of the cis configuration.

The conventional position for a double bond in other natural fats or oils composed of $C_{18}$ acids is $\Delta 9$ i.e. between carbon 9 and 10 of each of the fatty acids, but jojoba oil has mainly $\Delta 11$ and $\Delta 13$ unsaturation in the alcohols and acids, because of the large amount of $C_{20}$ and $C_{22}$ chains. All of the jojoba monoenes can be placed in the $\omega$-9 acids or $\omega$ 9 alcohols homologous series.

Jojoba oil is chemically more pure than most natural substances, 97 percent or greater liquid wax esters by gas chromatography. Yellow pigments in the oil can be removed with bleaching earths. The oil also has narrow range of wax-ester structures-over 83 percent $C_{20}$ and $C_{22}$ acids and alcohols.

Cis-to-trans-isomerization of the double bonds has been induced with selenium and nitrous oxide catalysts, producing up to 80 percent trans and 20 percent cis double bonds, The product is a soft solid with a melting point of about $44^O$C. The amount of such isomers formed can be controlled during these isomerizations by reaction time to obtain any melting point between that of the pure oil ($7^O$C) and $44^O$C. A product with a melting point close to that of the human body is desirable. However, both selenium and nitrous oxide previously used as catalysts present potential dermatological problems.

It is an object of this invention to provide a method for the cis-trans isomerization of jojoba oil to provide isomerates of jojoba oil having softening points in the range 25-40°C.

It is a further object to provide such isomerates by a process whereby the products directly are of sufficient purity for topical application.

Another object is to provide jojoba isomerates having a softening point at about or just below body temperature for use in formulations for topical application to the skin and at other temperatures for other uses.

There is also a need for solid wax-like materials (in contrast) to the semi-solid isomerates) having the useful properties of jojoba oils. Waxes of this type would be particularly useful to replace saturated animal, mineral or vegetable waxes from other sources in the preparation of special solid formulations for food, cosmetic, leather treating, or machine lubricating uses. Attempts have been made to use hydrogenated jojoba oil. Jojoba oil hydrogenate is a white, hard, crystalline material having a m.p. of 68-70°C. This material, like all high melting waxes, is very brittle at room temperature. Attempts have been made to modify jojoba oil hydrogenate by blending the hydrogenate with jojoba oil. When the oil and the hydrogenate are blended as by melting, the melting points of the resulting cooled compositions can be adjusted to within any desired temperature between the melting points of the two components. However on standing 3-5 days, the cooled compositions become grainy and the jojoba oil bleeds from the compositions, leaving behind the solid hydrogenate. The physical properties of such compositions are non-homogenous and thus undesirable. Similar grainy results are obtained by partially hydrogenating jojoba oils as the result is essentially a blend of the raw oil and the hydrogenate.

A further object is to provide compositions containing the isomerates suitable for leather treatment whereby the compositions containing the isomerate of jojoba oil may be incorporated into the hide or skin substrates at elevated temperatures in the substrate upon cooling to normal temperatures.

A further object is thus providing solid wax-like materials of varying physical properties similar to waxes but having the desired chemical properties and qualities of jojoba oil. Such compositions should be homogeneous and free from graininess and undesirable "bleeding ".

These and other objects will be readily apparent from the description of the process and the products derived therefrom which follows.

The present invention provides a method for the preparation of isomerates of jojoba oil melting above about 25°C which comprises the steps of contacting jojoba oil with an acidic, bentonite-type clay at temperature in the range 150-350°C for a time sufficient to isomerize the oil from the naturally occurring cis-configuration to the trans-configuration in amounts sufficient to provide an isomerate having a melting point in the range 25 to about 44°C.

The present invention is based on the discovery that acidic bentonite-type clays when contacted with jojoba oil initiate isomerization of the cis-configurations thereof to the transconfiguration above certain temperatures. At room temperature to about 150°C there is little or no isomerization upon contact, even over extended (24 hour )period of time. However, at temperatures above about 150°C the isomerization reaction begins to take place and accelerates upon increased temperatures. The isomerization can take place up to about 350°C.

The amount of isomerization of the oil may be followed by determining the melting point of the isomerate. Commercial jojoba oil melts at about 5-9°C. Refined jojoba oil, substantially pure cis-configuration, melts at 7°C. The pure transconfigured oil is reported to melt at about 54°C. As the percentage isomerization increases the melting point of the oil increases. At the conditions of isomerization of the invention, the melting point of such isomerized oil is in the range 25-30°.

The amount of isomerization with the clay is temperature dependent provided that sufficient clay is present to promote the reaction. Clay amounts as little as 0.005 weight % will cause isomerization at low rates. The amount of clay needed to initiate the reaction at a creditably rate is about 0.5 wt% based on the weight of the oil. Preferably about two wt% of the clay should be used to provide suitable isomerization rates at each temperature. Suitable isomerization rates are those wherein the melting point of the isomerizate at a given contact temperature stabilizes and becomes substantially constant after about half to one and one half hour of contact with the catalyst.

The degree of isomerization, as measured by the melting point of the isomerate varies with the contact temperature. At contact temperatures of about $200^{O}C$, unrefined oil, in contact with 2 wt% of the preferred clays for at least one and one half hours, provides materials melting in the range $27-28^{O}C$. At $220^{O}C$, the melting points range from about $29-30.5^{O}C$ and at $240^{O}C$ from about $30-31.5^{O}C$. Utilizing a refined jojoba oil, the melting points obtained are about two to three degrees higher.

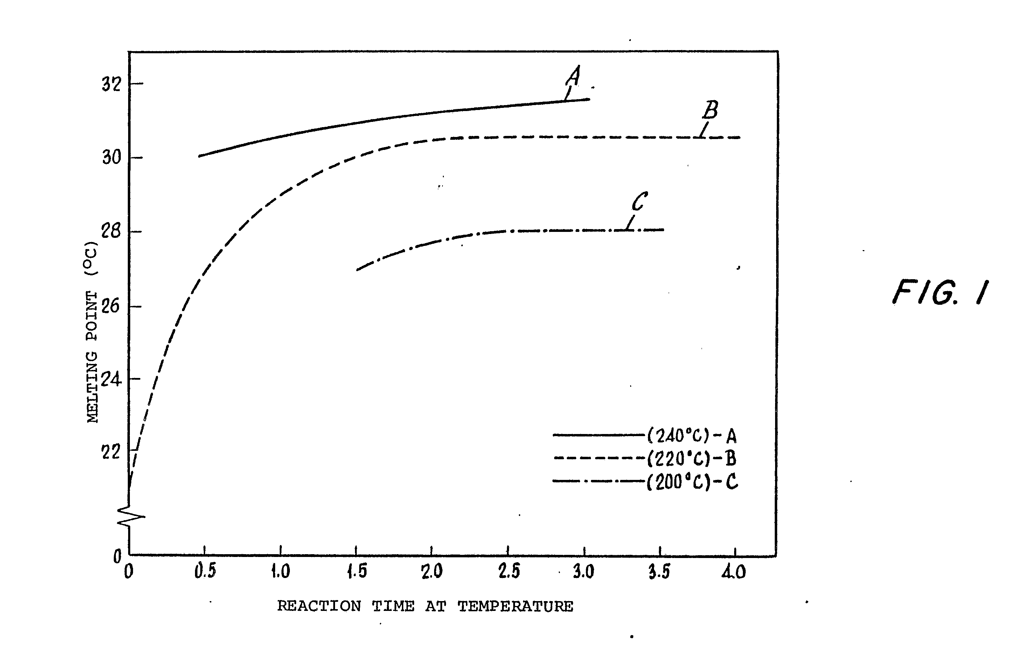

Figure 1 shows the curve demonstrating the relationship of the isomerate melting point as a function of the reaction contact time and the reaction temperature. The amount of clay used in the reaction mass was two wt% of the oil introduced. The melting points of the isomerate at various temperatures and times was determined by standard methods. It will be noted that the melting point stabilizes at each contact temperature and even prolonged contact with the clay at the temperature has little effect on the melting pint after the initial rise to stabilization of the melting point and consequent degree of isomerization.

The acidic bentonite-type clays that promote the isomerization at the stated temperature range are of the class that is well known for its use at lower temperatures for bleaching mineral and edible oils. These clays are of the montmorillonite crystalline form and have been treated with acid, usually HCl to modify the configuration and expand the total surface of the clay . Such acidic bleaching clays are generally used at temperatures up to about 125°C for bleaching oils. At higher temperatures no bleaching advantage has ever been noted and consequently the clays were not used at higher temperatures. There is no relationship between selenium isomerization catalyst structures and the present clays, nor any theoretical basis for concluding that selenium or nitrogen · oxides are either structurally or physically related in any manner to the acidic clays which are effective in the isomerization of jojoba oils.

Careful review of the clays effective for isomerization according to this invention shows that all have a montmorillonite crystallinity and have a pH of less than about 4.0 and preferably about pH 3.0. For convenience in later purification of the isomerized oil it is preferred that the acidic bleaching clay have less than about 0.25 wt% of residual acid. The pH and residual acidity are present as a result of the treatment of the clay with mineral acids to open the clay "books " and provide increased surface areas and exposure of internal crystalline bonding sites. While not wishing to the confined to any specific theory for the effectiveness of the specifically active clays, it is believed that the opening of the leaves of the crystalline clay "books" provides sites of suitable dimensions for the cis to trans reconfiguration.

As a result of the low pH of the clays and because of residual acidity of such clays, it has been found that the isomerates contain notable amounts of free acidity. As this is undesirable, it is preferable, after the desired degree of isomerization has been completed, to neutralize or remove this free acidity. This can be accomplished by contacting the isomerate, before or after removal of the suspended clay, with means for removal of such acidity. The isomerized oil may be contacted with solid basic substances such as NaOH, KOH, CaO, Ca(OH)$_2$, Na$_2$CO$_3$. Concentrated solutions of these basic substances , may also be used provided that the basicity and ionization are adjusted to prevent saponification of the wax to the respective alcohol and salt of the acid. Other free acid removal means are solid ion-exchange resins or zeolites in the basic phase. The basic components thereof react with the free acidity and the so-neutralized oil is filtered from the reacted resin.

As pointed out above, the amount of clay used is not critical, but sufficient clay should be present to initiate and support the isomerization reaction. Excess clay has no effect on the reaction rate or degree of isomerization, but too much clay may absorb excess oil and thus interfere with the overall yield. Amounts of the preferred clay may range from at least about 0.5 wt% up to about 40 wt% based on the amount of oil being treated. Preferably the amount of clay should be in the range from about 1 to 10 wt% with the optimal range being about 2 wt%.

The contacting of the oil with the clay can take place in any convenient vessel which can provide and maintain the required temperature range. As there is a tendency for jojoba oil to oxidize either at the points of unsaturation or esterification, especially in contact with the expanded surface areas provided by the treated clays, it is preferred to maintain the reaction mass of oil and clay under an inert or protective atmosphere. Preferred as the inert atmosphere is nitrogen gas. The reaction vessel should be provided with suitable closures and piping to maintain such an inert atmosphere.

Upon completion of the isomerization in the reaction mass by suitable passage of time at the required temperature, the reaction mass is cooled to about 100°C and the clay is separated from the transformed oil. Separation is preferably by filteration, but other separation means including centrifugation can be used.

The oil, after separation from the clay, can be treated for removal of the free acidity as mentioned above. In addition, any odorous components which may have developed can be steam-stripped by passing steam through the isomerized oil.

It has been found that if the jojoba oil is refined before the isomerization step, that the development of undesirable color is minimized. The preferred refining procedure involves heating the jojoba oil to about 200°C from one half to one and one half hours to coagulate and precipitate any proteinaceous material and denature any other impurities in the crude oil. These impurities may be filtered off. The oil is then decolorized by contacting with a decolorant. The decolorizing takes place at between 50 and 150°C. Suitable decolorants include neutral clays, acidic clays (including those which promote isomerization at temperatures above 150°C), activated aluminas and activated carbons. Prior to isomerization the decolorizing step provides a product having very little color. When the decolorized step is postponed until after completion of the isomerization, the decolorization by the above decolorants is virtually ineffective. The color materials which then develop after isomerization are not picked up by the decolorants and the resulting isomerized product is darker than the material from the preferred procedure. However, where color is not objectionable, as where the isomerized oil is used in highly pigmented materials or for dark leathers or where the isomerized material is to be further chemically reacted with sulphur compounds for the preparation of special lubricants, the darker isomerized materials, with or without decolorization, are adequate.

The isomerized materials prepared according to this invention are semi-soft solids having a room temperature consistency approximating butter. The melting points referred to supra and hereinafter are determined by methods appropriate for such materials. According to some testing standards, the terms "Hardening Point", "Softening Point", "Set Point" or "Congealing Point" are also used to describe this transition temperature. The method used for the Melting Point temperatures reported herein are according to EOA-Method H-1"Determination of Congealing Point".

By the aforesaid process, the controlled isomerization reaction is provided which affords a controlled conversion of the cis-configured oil to the transconfiguration. This isomerization reaction usually is permitted to proceed until cis-trans, isomerized mixtures having the consistency of butter result. These butter-like isomerate mixtures have liquifying points in the range 25-44°C and preferably about 27-35°C so that they are liquid upon contact with the skin. Mixtures having such melting points are desirable for certain cosmetic vehicles. In fact, they may serve as such vehicles. At present, the preferred isomerate mixture has a buttery consistency and liquifies at about 31°C.

This invention also includes the ancillary discovery of successful homogenous compositions of wax-like consistency of an isomorphorous system of jojoba oil hydrogenate and the transisomerate of jojoba oil in mutual solid solution. Blends of various proportions of these isomorphous components can be prepared that are·stable solids.

Because of their isomorphous nature, these solid solutions do not evidence any eutectic condition but the compositions show increasing melting points with increases in the amount of jojoba hydrogenate in the compositions. Thus the invention also provides solid, waxy compositions entirely of jojoba-derived compounds and having useful melting points. They do not separate as they are in a homogenous condition.

The jojoba oil hydrogenate used for the preparation of these compositions is prepared and has the physical properties as described in the 1975 National Academy of Science report entitled : "Products From Jojoba: A Promising New Crop For Arid Lands." Details of the preparation are outlined therein (pg. 20ff.). The jojoba oil partial hydrogenates mentioned therein are not the creamy products postulated but rather are grainy mixtures of the cis-oriented liquids and the solid hydrogenate.

The buttery trans-isomerized jojoba oil used for the preparation of the composition of this invention are described and prepared by the isomerization process of this invention described above. These isomerates have the trans-configuration in contrast cis-configuration of the natural jojoba oil. These trans-isomerates have liquefying points in the range 25-44OC and preferably for the purposes of this invention liquefy at about 31-35°C and are preferably prepared by contacting the cis-configurated natural jojoba oil with an acidic-clay at temperatures in the range 150-350$^{\circ}$C until the desired degree of isomerization is achieved.

The combined waxy compositions of this invention are mutual solid solutions of their components, the isomerates and the hydrogenate. At low melting range, they can be prepared by dissolving the hydrogenate in the softened isomerate. However, it is preferred to heat the components to melting to speed solution. At the upper ranges of melting points of the isomorphous compositions of this invention they can be rapidly prepared by mixing the melts of the two components.

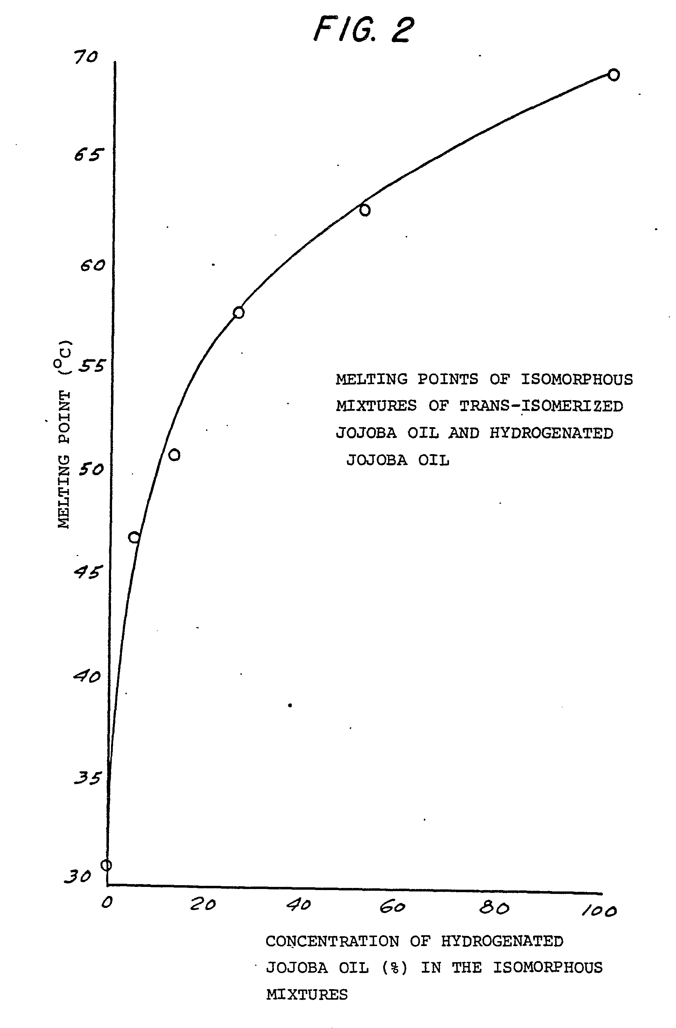

Figure 2 shows the variation of melting points with the combined isomerate/hydrogenate compositions. As can be seen, there is a smooth increase in the melting points with increases in hydrogenate content.

The compositions having 5, 12.5 and 25% hydrogenate contents have been formulated into creams, lip-sticks, lip balms, candles and fry-lubricants as replacements for the usually used waxes and performed as well or better as replacements therefore.

When molded into a lipstick, the compositions containing 12.5% hydrogenate retain good strength and do not soften appreciably in contact with body heat. No shattering or dis-figuration occurs when such lip-sticks in metal cases are subjected to standard 1.5 meter drop-tests to demonstrate brittleness.

Additionally, the stability of both components of the compositions and the compositions themselves to heat, light and oxidation, and general lack of toxicity makes the compositions of this invention particularly useful for candles, cosmetics, and in foods. In cosmetics, the compostions replace waxes as in lipsticks and creams. They have excellent emollient qualities when properly formulated in creams. In lip-sticks they raise the melting points without embrittlement and yet do not interfere with the smoothness of application. The compositions are compatible with the oils, sterols, detergents and dyestuffs commonly used in cosmetics. The compositions also are useful as components in hair laquers , dressings and shampoos and as "foots" in the treatment of leathers or in shoe-shine compositions.

In the food industry, the compositions of this invention are particularly useful in lubricant compositions for food machinery, as replacements for bread-pan greases, and as fat free fry-pan lubricant sprays. In the latter uses the compositions of this invention are dissolved in a non-toxic, low boiling solvent, injected into an aerosol can and then a propellant is introduced. The solvent is usually ethanol and the propellant is selected from among hydrocarbons, fluorocarbons and carbon dioxide.

As can be seen from the Figure 2, the addition of as low as 5% of hydrogenate to the isomerate, results in a melting point elevation of 16°C relative to the melting point of the isomerate.

As mentioned above, the present invention affords solid products from the semi-solid isomerate with additions of as little as 1% of hydrogenated jojoba oil.

Experiments A and B below provide comparisons of the qualities of the isomerate/hydrogenate mutual solution compositions of this invention under conditions for cosmetic and candle use as compared to mixtures of melts of the hydrogenate and jojoba oil.

Experiment A.

Comparison of mixes of 1.) Jojoba oil and Hydrogenated Jojoba Oil, with 2.) Jojoba Isomerate and Hydrogenated Jojoba Oil for cosmetic and lubricant purposes.

1. Hydrogenated Jojoba Oil is mixed well with Jojoba Oil at concentrations of Hydrogenate in Oil of 2.5% to 75% at temperatures in excess of 70°C when both materials are liquid. The resultant mixtures are allowed to cool to room temperature (25°C) in jars (approximately 2.54 cm in diameter and 1.27 cm high) and in cylinders (approximately 3.81 cm high and 0.95 cm in diameter). The resultant materials are examined for physical appearance, strength and consistency. In all cases the mixes are grainy in appearance and feel, with obvious bleeding of liquid oil occurring at all Hydrogeneate concentrations (2.5% to 75%).The mixes at very low hydrogenate concentrations (2.5% to 25%) are slushy and at up to 50% exhibit poor strength on impact. Lip- sticks made of mixes containing up to 25% hydrogenate concentrations tend to shatter or deform when dropped from heights of 1.5 meter in standard metal cases. Deformation occurs after short periods at body temperature (37°C).

2.      The experiment is repeated but this time using mixtures of hydrogenated jojoba oil and jojoba isomerate. The resulting mixtures have a consistency and strength similar to commercially available waxes used for lip-sticks, lip balms, cosmetic creams and food equipment lubricants.

Experiment B.

Comparaison of mixes of 1.) Jojoba Oil and Hydrogenated Jojoba Oil, with 2.) Jojoba Isomerate and Hydrogenated Jojoba Oil for Candles.

1.      The experiment A.1) is repeated but at hydrogenate concentrations up to 75% and the products are cast into candles approximately 12.7 cm long and 1.27 cm in diameter. In this range the products produced ar not suitable for use as a candle material because of oil bleeding. At low hydrogenate concentrations, a grainy, low strength , slushy appearance manifests itself. At higher concentrations the product is hard and brittle with oil bleeding occurring. The candles do not burn evenly but sputter badly.

2.      The experiment is repeated but using hydrogenate and the isomerate (31°C). At hydrogenate concentrations ranging from 25% to 85% material suitable for candles is produced. The hardness and burning rates are uniform. No evidence of oil bleeding is found.

The preferred modes according to the isomerization aspects of the present invention are described in appended examples 1-5. Each mode is preferred for the specific product described. The products which result from each modification are for specific and different applications and thus the different modes are preferred for the different products.

Example 1:

Commercial jojoba oil (MP-5-3°C) 100 gms is heated to 100°C and decolorized with 1 wt% of a substantially neutral (pH-6) bleaching clay (Tonsil L-8) for one hour. The oil is heated with agitation under nitrogen to 240°C and admixed with 2 gms. of finely ground acid-treated montmorillonite clay (Tonsil Optimum Extra FF manufactured by Tonsil Mexicana SA). The clay has a pH of 3.5-3.0 and between 0.018 to 0.225% free residual acid. The reaction mass is stirred and maintained at the isomerization temperature of 240°C. Melting points are run on samples extracted from the mix every 0.5 hours. Curve A in the Figure shows that the isomerization is rapidly initiated and that the degree of isomerization reflected in the elevated melting points stabilizes in the range 30-32°C within 0.5 to 3.0 hours. The reaction mix is then cooled to 100°C and the aciditic-clay is filtered from the mix. The oil isomerized having a melting point of 31.5°C, and Gardner color of about 7, is then contacted with powdered soda ash (Na$_2$CO$_3$) to neutralize any free acidity of the isomerate of jojoba oil.

Example 2

The procedure of Example 1 is repeated, but at an isomerization temperature of 220°C. Curve B of the Figure shows the melting points obtained during the course of the reaction.

Example 3

The procedure of Example 1 is repeated at an isomerization temperature of 200°C. Curve C shows the melting points obtained during the course of the reaction.

Example 4

The jojoba oil is refined before decolorization by heating the commercial oil to 100 - 200°C to coagulate the protein and lipid impurities. The heated mass is then decolorized and the isomerization procedures of Examples 1-3 are repeated.

The melting point curves obtained are similar to those of Figure 2, but uniformly displaced to reflect a 2.5 - 3.0°C elevation in melting point of the isomerate of the refined jojoba oil.

Example 5:

Commercial jojoba oil, 227 kg is introduced into an agitated, stainless steel jacketed vessel under a nitrogen atmosphere and heated to 200°C. After one hour at 200°C, one percent (2.27 kg) of powdered decolorizing or bleaching clay (Tonsil L-80-mfg by Tonsil Mexico SA) is introduced into the oil and mixed therewith for one hour. The clay and coagulum from the initial heating is then removed by filtration. The filtrate is then admixed with 2wt% i.e. 4.54 kg of the acid-treated montmorillonite clay (pHS) (Tonsil Optimum or Tonsil Optimum Extra, or Tonsil Optimum FF, differing from each other only in residual acidity and particle size) and the resulting mass is heated to 240°C for 3 hours until isomerization of the mass, as determined by the melting point of the successive abstracted samples, stabilizes. The reaction mass is cooled to 100°C and the isomerizing clay is filtered off. The heated filtrate is conducted through a bed of powdered $Na_2CO_3$ of sufficient depth to remove any residual acidity from the isomerized oil. The neutralized oil, upon cooling to room temperature, had a light color (Gardner No. 5-6) and a semi-soft consistency of butter. The M.P. of the product is 34-35°C. The product is suitable for direct topical application as a moisturizing and lubricating cream, as well as for formulation into various cosmetic formulations where such a high melting oil having the characteristic stability of jojoba oil is indicated.

The isomerized jojoba "butter" is also suitable for the direct treatment of leather to improve the suppleness. The isomerized oil also reacts with sulphur compounds in a manner analagous to the cis-isomer oils to provide lubricants suitable for use at extreme temperatures and pressures.

The examples 6-17 which follow illustrate representative cosmetic and other formulations utilizing the compositions of this invention. The trademarked designation, Jojobutter, as used herein refers to the isomerate, isomorphous compositions containing same, including those according to this invention containing the isomerate and hydrogenate of jojoba oil in isomorphous mixture. The number following the trademark term Jojobutter refers to the nominal melting, liquifying or softening point in degrees Celsius of the specific composition and indicates its "melting" grade.

The compositions according to this invention may replace or be combined with other waxes for specific properties. When the compositions are used to replace known hard waxes it is useful to substitute a melting grade that approximates the melting point of the wax being replaced.

The jojoba oil mentioned in the formulae is the natural refined cis-oil.

The basic cosmetic formulae, as herein modified, are from the Formulators Handbook published in 1980 by Amerchol Coro. and the trademarks mentioned herein are those of products of the publisher or are more specifically identified in said handbook as to source. The generic terms for the components therein used are those designated by the CFTA of approved and recognized products.

### Example 6

#### Lipstick

| | |
|---|---|
| Castor Oil | 49.0% |
| Acetulan | 4.0 |
| Beeswax | 9.0 |
| Jojobutter -31 | 10.0 |
| Isopropyl Palmitate | 11.0 |
| Ozokerite | 5.0 |
| Jojobutter 63 | 5.0 |
| Color and perfume | q.s. |

### Example 7

#### Sun Protection Lotion

OIL PHASE

| | |
|---|---|
| Amerchol-101 | 4.2% |
| Mineral oil 70 wt. | 10.2 |
| Acetulan | 2.5 |
| Isopropyl myristate | 8.5 |
| Glyceryl stearate | 4.2 |
| Laureth-23 | 4.2 |
| Jojoba oil | 0.5 |
| Jojobutter-51 | 2.5 |
| Amerscreen P | 3.0 |

WATER PHASE

| | |
|---|---|
| Water | 40.8% |
| Polysorbate 20 | 1.7 |
| Carbomer 934, 3% slurry | 8.5 |
| Triethanolamine, 10% | 9.2 |

Perfume and preservative          q.s.

### Example 8

#### Sun Tan Oil

Vegetable oil based lotion . Good moisturizer.

| | |
|---|---|
| Amerscreen P | 3.0 % |
| PPG-Myreth-3 | 30.0 |
| Acetulan | 5.0 |
| Amerchol-101 | 5.0 |
| Ameroxol OE-2 | 5.0 |
| Diisopropyl adipate | 1.0 |
| Octyl palmitate | 4.0 |
| Isodecyl oleate | 1.0 |
| Jojoba oil | 5.0 |
| Jojobutter-31 | 10.0 |
| Sesame oil | 31.0 |
| Perfume | q.s. |

### Example 9

#### Sun Protection Stick

Hard anhydrous butter with emollient properties.

| | |
|---|---|
| Amerscreen P | 1.5% |
| PPG-3-Myreth-3 | 5.0 |
| Acetulan | 3.0 |
| Amerchol CAB | 20.0 |
| Petrolatum | 20.0 |
| Cocoa butter | 10.0 |
| Modulan | 10.0 |
| Mineral oil | 10.0 |
| Jojoba oil | 6.0 |
| Olive oil | 6.0 |
| Sesame oil | 4.5 |
| Jojobutter-63 | 4.0 |
| Perfume | q.s. |

### Example 10

#### Sun Protection Cream

Soft butter with skin treatment properties.

| | |
|---|---|
| Amerscreen P | 1.5 % |
| PPG -3-Myreth -3 | 5.0 |
| Acetulan | 3.0 |
| Modulan | 10.0 |

| Amerlate P | 3.0 |
|---|---|
| Jojoba oil | 7.0 |
| Sweet Almond oil | 7.0 |
| Jojobutter-51 | 10.0 |
| Microcrystalline wax | 3.0 |
| Mineral oil | 10.5 |
| Amerchol CAB | 20.0 |
| Petrolatum | 20.0 |
| Perfume | q.s. |

### Example 11

### Soft Sun Cream

Soft butter suitable for tube dispensing with emollient properties and easy spreading.

| Amerscreen P | 1.5% |
|---|---|
| PPG -3-Myreth-3 | 5.0 |
| OHlan | 5.0 |
| Microcrystalline wax | 5.0 |
| Octyl palmitate | 8.5 |
| Jojobutter-31 | 10.0 |
| Microcrystalline wax | 15.0 |
| Amerchol-101 | 40.0 |
| Mineral oil | 10.0 |
| Perfume | q.s. |

### Example 12

Nongreasy cream. Designed to provide maximal sun protection:

OIL PHASE

| Jojoba oil | 7.0% |
|---|---|
| Amerchol CAB | 2.0 |
| Jojobutter-51 | 5.0 |
| Glyceryl stearate | 8.5 |
| Amerscreen P | 5.0 |
| Glucate | 1.0 |
| Glucamate SSE-20 | 1.0 |

WATER PHASE

| Water | 67.5 |
|---|---|
| Glucam E-10 | 2.0 |
| Triethanolamine | 1.0 |
| Perfume and preservative | q.s. |

Example 13

Soft, elegant, nonionic cream, nongreasy, Excellent rub-in:

OIL PHASE

| | |
|---|---|
| Jojoba oil | 5.0% |
| Solulan PB-20 | 3.0 |
| Jojobutter -51 | 5.0 |
| Myristyl alcohol | 2.0 |
| OHlan | 1.0 |
| Glucate SS | 3.0 |
| Glucamate SSE-20 | 3.0 |
| Acetulan | 0.5 |
| Amerscreen P | 3.0 |
| Arlacel 165 | 5.0 |
| Amerchol L-101 | 10.0 |

WATER PHASE

| | |
|---|---|
| Water | 58.0 |
| Glucam E-20 | 1.5 |
| Perfume and preservative | q.s. |

Example 14

Candle Wax

| | |
|---|---|
| Jojobutter-63 | 80% |
| Microcrystalline wax | 20 |

Example 15

Candle Wax

| | |
|---|---|
| Jojobutter -63 | 90.0% |
| Polyethylene | 1.0 |
| Petrolatum | 9.0 |

Example 16

Pan Release (acrosol)

| | |
|---|---|
| Jojobutter- 51 | 10% |
| Ethanol | 90 |

The partial solution of the Jojobutter was introduced into an aerosol can. The pressurized propellant was then introduced to complete the solution. Applied as a spray to bake pans and fry pans, it provided excellent release of the baked goods from the bake pans and reduced adherence of residual foods to the fry pans.

Example 17

Pan Releases (non-aerosol)

| | |
|---|---|
| Jojobutter - 47 | 24 % |
| Corn oil | 10 |
| Emulsifier (Food Grade) | 5 |
| Water | q.s. |

The emulsion is prepared in a homogenizer. The concentrate is bottled with directions for dilution. It is diluted with more water and sprayed unto the pan before frying or baking.

CLAIMS:

1.       A method for the preparation of isomerates of jojoba oil melting above about 25°C characterized in that it comprises the steps of contacting jojoba oil with an acidic-bentonite-type clay at temperatures in the range 150-350°C for a time sufficient to isomerize said oil to provide an isomerate having a melting point in the range 25 to at least about 44°C.

2.       The method according to claim 1, characterized in that said clay is a comminuted montmorillonite, expanded and acid-treated, having a pH less than about 4 but with less than about 0.25 wt % residual acid.

3.       The method according to claim 1, characterized in that the oil is contacted with from about 0.005 to about 40 wt % of said clay.

4.       The process according to claim 1, characterized in that said contacting is conducted in an inert atmosphere.

5.       The process according to claim 1, characterized in that said atmosphere is a nitrogen atmosphere.

6.       The process according to claim 3, characterized in that the amount of said clay ranges from about 0.5 to about 5 wt % of said oil.

7.       The process according to claim 6, characterized in that said amount is about 2 wt% of said oil.

8.       The process according to claim 1, characterized in that an isomerate having a melting point of about 20-29°C is obtained by contacting the oil with about 2 wt% of an acid -treated montmorillonite clay having a pH of about 3-3.5 at a temperature of about 200°C for about at least 1.0 hours.

9.       The process according to claim 1, characterized in that an isomerate having a melting point in the range 27-30°C is obtained by contacting the oil with about 2wt% of an acidic montmorillonite clay having a pH of 3-3.5 for about 1.0 hour at 220°C.

10.      The process according to claim 1, characterized in that an isomerate having a melting point of about 30-35°C is obtained by contacting the oil with about 2 wt% of an acidic montmorillonite clay having a pH of about 3-3.5 for at least 0.5 hours at a temperature about 240°C.

11. The process according to claim 1, characterized in that the jojoba oil, before isomerization is refined by heating the oil to about 200°C for about 1 hour to coagulate and precipitate proteins and other lipid impurities.

12. The process according to claim 11, characterized in that said refining also includes contacting said oil with at least one decolorant selected from the group consisting of neutral pH bleaching-clay, activated-aluminas and activated-charcoal.

13. The process according to claim 11, characterized in that said refining includes decolorizing said oil, after coagulation and precipitation, by contacting said oil with a neutral -pH bleaching bentonite clay.

14. The process according to claim 1 characterized in that the reaction mass of oil and clay after completion of the isomerization time is cooled to about 50-100°C, and filtered to remove the said clay.

15. Theprocess according to claim 14, characterized in that the reaction mass before filtration is neutralized by contact with a basic substance in powder or solution form to reduce any free-acidity in the isomerate.

16. The process according to claim 14, characterized in that said isomerate, after removal of said clay, is steam-stripped of odorous impurities.

17. The product , an isomerate of jojoba oil characterized in having a melting point in the range 25-44°C prepared by the process according to claim 1.

18. The product according to claim 17, characterized in that said isomerate has a melting point in the range 25-40°C and is free from selenium, nickel, sulphur or nitrogen-oxide catalytic residues.

19. A homogenous composition consisting esentially of trans-isomerized jojoba oil according to claim 1 and hydrogenated jojoba oil in the isomorphous state of mutual solution.

20. The composition according to claim 19, characterized in that the isomorphous solution is in the solid state.

21. The composition according to claim 19, characterized in that said hydrogenate comprises five to ninety-five percent of said composition.

22. The composition according to claim 19, characterized in that the trans-isomerized jojoba oil component of said composition has a liquifying point in the range about 28-35°C.

23. The composition according to claim 22, characterized in that said isomerized component has a liquifying point of about 31°C.

24. The composition according to claim 19, as a wax substitute in cosmetic formulations.

25. The composition according to claim 19 as a solidifying wax component in lipsticks.

26. The composition according to claim 19 as a component in candle wax.

27. The composition according to claim 19 as the functionally active component in non-stick sprays for fry and baking pans.

28. The composition according to claim 19 as the wax component in the shoe polishes and waxes.

29. The composition according to claim 19 as a wax component of textile sizes.

30. The composition according to claim 19 as a carrier component in detergent "soaps" shampoos and hair conditioners.

31. The composition according to claim 19, characterized in that said composition comprises an enteric coating wax for tablets and pills.

32. The composition according to claim 19, characterized in that said composition is utilized as a component of a food machinery lubricant.

FIG. 1

MELTING POINT (°C) vs REACTION TIME AT TEMPERATURE

(240°C) – A
(220°C) – B
(200°C) – C

0046723

# FIG. 2

MELTING POINTS OF ISOMORPHOUS
MIXTURES OF TRANS-ISOMERIZED
JOJOBA OIL AND HYDROGENATED
JOJOBA OIL

CONCENTRATION OF HYDROGENATED
JOJOBA OIL (%) IN THE ISOMORPHOUS
MIXTURES

0046723

European Patent Office

**EUROPEAN SEARCH REPORT**

Application number

EP 81 63 0049

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | INDUSTRIAL AND ENGINEERING CHE-MISTRY. PRODUCT RESEARCH AND DEVELOPMENT, vol. 14 (3), 1975, Washington, US J. WISNIAK et al.:"Geometrical Isomerization of Jojoba Oil", pages 177-180 <br> * Pages 177, 178 * | 1 | C 11 C 3/14 <br> A 61 K 7/00 <br> 9/42 |
| | DE - B - 1 211 158 (HENKEL) <br> * Claim; page 3, lines 1-10; example 1 * | 1-7 | |
| | | | **TECHNICAL FIELDS SEARCHED (Int. Cl.³)** |
| X | JOURNAL OF THE AMERICAN OIL CHE-MISTS' SOCIETY, vol. 57,(3)1980 Champaign Illinois, US A. SHANI et al.: "Synthesis of Jojobamide and Homojojobamide" pages 112-114 <br> * Page 112, column 1,experimental procedures, general * | 12 | C 11 C 3/14 |
| | | | **CATEGORY OF CITED DOCUMENTS** <br> X: particularly relevant <br> A: technological background <br> O: non-written disclosure <br> P: intermediate document <br> T: theory or principle underlying the invention <br> E: conflicting application <br> D: document cited in the application <br> L: citation for other reasons <br> &: member of the same patent family, corresponding document |

| | | |
|---|---|---|
| The present search report has been drawn up for all claims | | |
| Place of search <br> The Hague | Date of completion of the search <br> 01-12-1981 | Examiner <br> PEETERS J |

EPO Form 1503.1  06.78